# NOUVEAU FASCICULE DE BREVET EUROPEEN

(11) **EP 0 722 722 B2**
(45) Date de publication et mention de la décision concernant l'opposition: **05.03.2003**
(45) Mention de la délivrance du brevet: 23.09.1998
(21) Numéro de dépôt: 95402582.1
(22) Date de dépôt: 17.11.1995
(51) Int. Cl.: A61K 31/00, A61K 38/08, A61P 17/00

(54) **Utilisation d'un antagoniste de substance P pour le traitement des rougeurs cutanées d'origine neurogène**
Verwendung eines Antagonisten der Substanz P zur Behandlung von Hautrötungen neurogen Ursprungs
Use of an antagonist of substance P for the treatment of cutaneous erythema of neurogenic origin

(30) Priorité: 19.12.1994 FR 9415252
(43) Date de publication de la demande: 24.07.1996
(73) Titulaire: L'OREAL, 75008 Paris (FR)
(72) Inventeur: De Lacharriere, Olivier, F-75015 Paris (FR); Breton, Lionel, F-78000 Versailles (FR)
(74) Mandataire: Lhoste, Catherine

(56) Documents cités:
- EP-A- 0 360 390
- EP-A- 0 520 555
- EP-A- 0 522 808
- EP-A- 0 577 394
- EP-A- 0 680 749
- WO-A-93/01159
- WO-A-93/14084
- GB-A- 2 271 774
- BRITISH JOURNAL OF PHARMACOLOGY, vol. 106, no. 2, 1992, pages 256-262, XP002000816 YONEHARA, N. ET AL: "Involvement of substance P in primary afferent neurones in modulation of cutaneous blood flow in the instep of rat hind paw"
- EUROPEAN JOURNAL OF PHARMACOLOGY, vol. 85, 1982, pages 171-176, XP002000817 LEMBECK, F. ET AL: "Inhibition of neurogenic vasodilation and plasma extravasation by substance P antagonists, somatostatin and (D-met2,pro5)enkephalinamide "
- SCIENCE (WASH D C), 214 (4524). 1981. 1029-1031., XP002000818 HOLMDAHL G ET AL: "A SUBSTANCE P ANTAGONIST 2-D PROLINE 7 9-D-TRYPTOPHAN SUBSTANCE P INHIBITS INFLAMMATORY RESPONSES IN THE RABBIT EYE"
- ANN. INTERN. MED., 1981, 95/4 (468-476), USA, XP002000819 WILKIN J.K.: "Flushing reactions: Consequences and mechanisms"
- AGENTS ACTIONS, 1983, 13/2-3 (105-116), SWITZERLAND, XP002000820 FOREMAN J. ET AL: "Histamine release and vascular changes induced by neuropeptides"
- BRITISH JOURNAL OF PHARMACOLOGY, vol. 109, no. 1, 1993, pages 259-264, XP002000821 MOUSSAOUI, S.M. ET AL: "A non-peptide NK1-receptor antagonist, RP 67580, inhibits neurogenic inflammation postsynaptically"
- BR J PHARMACOL, DEC 1993, 110 (4) P1614-20, ENGLAND, XP002000822 INOUE H ET AL: "Profile of capsaicin-induced mouse ear oedema as neurogenic inflammatory model: comparison with arachidonic acid-induced ear oedema."
- BRITISH JOURNAL OF PHARMACOLOGY, vol. 105, no. 3, 1992, pages 527-530, XP002000823 LEMBECK, F. ET AL: "The non-peptide tachykinin antagonist, CP-96,345, is a potent inhibitor of neurogenic inflammation"
- Pschyrembel, Klinisches Wörterbuch, 256.Auflage, S.415-417; 477; 1750
- Eedy DJ Brit.J.Dermatol.(1993) 128, 597-605
- "Dermatologie und Venerologie", 3.Auflage Springer Verlag, 1984(textbook)
- Rebora A, Clinics in Dermatology (1993)11,324-328
- Wilkin JK, Ann.Intern.Med (1981) 95, 468-476
- Wallengreen J, Br.J.Dermatol. (1991) 124, 324-328
- Lowe JA,DN&P (1992),5(4), 223-228
- Moussaoui SM et al., Br.J.Pharmacol.(1993) 109, 259-264
- Holmdahl G et al, Science (1981) 214, 1029-1031
- Lembeck F et., Br.J.Pharmacol.(1992)105,527-530
- Lembeck F et al., Eur.J.Pharmacol.(1982)85, 171-176
- Dubertret L Thérapeutique Dermatologique (1991) 529-530
- Textbook of Dermatology, Rook/Wilkinson/Ebling, sixth edition 1998,Chapter 46: Flushing ad Flushing Syndromes, Rosacea, p.2104-2111
- Gastroenterology (1993) 104
- Effet du SrCl2 quantifié par thermographie ( note T 29.10.01 )
- Br.J.Dermatol.(1982)107(1), 59-61

## Description

La présente invention se rapporte à l'utilisation d'un antagoniste de substance P pour la préparation d'une composition topique pharmaceutique pour traiter la rosacée.

La rosacée est une affection cutanée caractérisée par un érythème du visage prédominant sur les pommettes, le front, le nez, une hyperséborrhée du visage au niveau du front, du nez et des joues, et par une composante infectieuse avec des pustules acnéiformes.

Par ailleurs, il s'associe à ces signes une composante neurogène, c'est-à-dire une hyperréactivité de la peau du visage et du cou, caractérisée par l'apparition de rougeurs et de sensations subjectives du type démangeaisons ou prurit, de sensations de brûlures ou d'échauffement, de sensations de picotements, de fourmillements, d'inconforts, de tiraillements, etc.

Ces signes d'hyperréactivité peuvent être déclenchés par des facteurs très divers tels que la prise d'aliments, de boissons chaudes ou alcoolisées; par des variations de température rapide, par la chaleur et notamment l'exposition aux ultraviolets ou aux infrarouges, par une humidité relative basse, par l'exposition de la peau aux vents violents ou aux courants d'air (soufflerie, air conditionné), par l'application de tensio-actifs, de topiques dermatologiques irritants (rétinoïdes, peroxyde de benzoyle, alpha-hydroxy-acides...) ou l'utilisation de certains cosmétiques même lorsque ceux-ci ne sont pas connus comme particulièrement irritants.

Jusqu'alors, le mécanisme de déclenchement de ces signes était très mal connu et la rosacée était traitée par voie topique par des actifs tels que les antiséborrhéiques et les anti-infectieux, par exemple le peroxyde de benzoyle, l'acide rétinoïque, le métronidazol ou les cyclines, qui agissaient sur l'infection et l'hyperséborrhée, mais ne permettaient pas de traiter la composante neurogène de cette affection, et notamment l'hyperréactivité de la peau et la rougeur.

De même, il n'existait pas jusqu'à présent de traitement topique pour la rougeur apparaissant dans l'érythème pudique. Celui-ci survient lors d'une émotion et se caractérise par une rougeur du visage et du décolleté, qui peut éventuellement s'accompagner d'un prurit (démangeaisons). Cette affection est très gênante pour les personnes qui en souffrent, et jusqu'alors on ne pouvait la traiter que par des bêta-bloquants, médicaments puissants utilisés pour traiter l'hypertension et présentant de nombreuses contre-indications.

Il subsiste donc le besoin d'un traitement, par voie topique, de la rougeur cutanée, composante essentielle de cet état d'hyperréactivité de la peau atteinte de rosacée ou d'érythème pudique.

La présente invention a justement pour objet l'utilisation d'un ou plusieurs antagonistes de substance P pour traiter ces affections par voie topique.

La substance P est un élément chimique polypeptidique élaboré et libéré par une terminaison nerveuse. Elle fait partie de la famille des tachykinines qui proviennent des terminaisons nerveuses libres de l'épiderme et du derme. La substance P intervient notamment dans la transmission de la douleur et dans des maladies du système nerveux central tels que l'anxiété, la schizophrénie, dans des maladies respiratoires et inflammatoires, dans des maladies gastro-intestinales, dans des maladies rhumatismales et dans certaines maladies dermatologiques telles que l'eczéma, le psoriasis, l'urticaire et les dermites de contact.

Il est connu d'utiliser les antagonistes de substance P pour traiter les maladies indiquées ci-dessus. A cet effet, on peut se référer aux documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569, GB-A-2216529, EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116, EP-A-522808, WO-A-93/01165, WO-A-93/10073 et WO-A-94/08997.

Par ailleurs, certains documents, et notamment J. K. Wilkin, Annales of Internal Medicine, 1981, vol. 95, p.468-476, enseignent l'intervention de la substance P dans l'afflût de sang sur le visage. Toutefois, ces documents ne décrivent ni ne suggèrent d'utiliser un antagoniste de substance P par voie topique pour éviter les rougeurs cutanées d'origine neurogène.

Aussi, la présente invention a pour objet l'utilisation selon la revendication 1.

L'application de compositions contenant un ou des antagonistes de substance P permet d'obtenir une nette diminution voire une disparition complète de la rougeur qui se manifeste aussi bien dans la rosacée que dans l'érythème pudique.

L'antagoniste de substance P agit donc sur la composante neurogène de ces affections, pour laquelle on n'avait pas de traitement jusqu'à présent, et renforce ainsi l'efficacité des actifs utilisés jusqu'à présent pour le traitement de leur composante infectieuse, notamment dans le cas de la rosacée.

La composition de l'invention contient un milieu pharmaceutiquement ou dermatologiquement acceptable, c'est-à-dire un milieu compatible avec la peau, les ongles, les muqueuses, les tissus et les cheveux. La composition contenant l'antagoniste de substance P peut être appliquée par voie topique sur le visage, le cou, les cheveux, les muqueuses et les ongles, les grands plis ou toute autre zone cutanée du corps.

Pour qu'une substance soit reconnue comme un antagoniste de substance P, elle doit répondre à la caractéristique suivante :
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

Lantagoniste de substance P peut en outre avoir une affinité sélective pour les récepteurs NK1 des tachykinines.

L'antagoniste de substance P de l'invention peut être fonctionne ou réceptoriel, c'est-à-dire inhiber la synthèse et/ou la libération de substance P, ou empêcher sa fixation et/ou moduler son action.

L'antagoniste de substance P de l'invention peut être choisi parmi les peptides ou les dérivés non peptidiques, et plus précisément ceux comportant un hétérocycle azoté, soufré ou oxygéné, ou les composés azotés comportant un atome d'azote lié directement ou indirectement à un cycle benzénique

On peut utiliser dans l'invention par exemple comme peptide antagoniste de substance P le sendide et le spantide Il.

Le sendide correspond à la formule :
Tyr D-Phe Phe D-His Leu Met NH₂
dans laquelle :
Tyr représente la tyrosine,
D-Phe représente la D-phénylalanine,
Phe représente la phénylalanine,
D-His représente la D-histidine,
Leu représente la leucine,
Met représente la méthionine.

Le spantide II correspond à la formule :
D-NicLys Pro 3-Pal Pro D-CI₂Phe Asn D-Trp Phe D-Trp Leu Nle NH₂
dans laquelle :
D-NicLys représente.le nicotinate de D-lysine,
Pro représente la proline,
3-Pal représente la 3-pyridyl-alanine
D-CI₂Phe représente la D-dichlorophénylalanine,
Asn représente l'asparagine,
D-Trp représente le D-tryptophane,
Phe représente la phénylalanine,
Leu représente la leucine,
Nle représente la nor-leucine.

On peut également utiliser dans l'invention comme peptide antagoniste de substance P les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés hétérocycliques, notamment azotés, soufrés ou oxygénés, ou des composés comprenant un atome d'azote lié directement ou indirectement à un cycle benzénique.

Comme composé hétérocyclique, on peut utiliser dans l'invention ceux comportant un hétérocycle azoté décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116, WO-A-94/08997. En particulier, le composé comprenant au moins un hétérocycle azoté est un dérivé de 2-tricydyl-2-amino-éthane, un dérivé de spirolactame, un dérivé de quinuclidine, un dérivé azacyclique, un dérivé d'aminopyrrolidine, un dérivé de pipéridine, un aminoazahétérocycle, un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy- tétrazolyl-benzofuranne-carboxamides ou les alcoxy- et/ou aryloxy- tétrazolyl-benzothiophène-carboxamides.

Comme composés comportant un atome d'azote lié directement ou indirectement à un noyau benzénique, on peut citer ceux décrits dans les documents suivants : EP-A-522808, WO-A-93/01165 et WO-A-93/10073. On peut citer notamment les dérivés d'éthylène diamine, tels que la N,N'-bis-di-(3,5-dimethylbenzyle)- éthyléne diamine, la N,N'-bis-di-(3,5-dimethoxybenzyle)-éthylène diamine ces composés sont décrits comme intermédiaires de synthèse dans le document WO-A-93/11338 déposé au nom de la demanderesse.

Les antagonistes de substance P peuvent être synthétisés ou extraits de produits naturels (végétaux ou animaux).

Dans les compositions selon l'invention, l'antagoniste de substance P est utilisé de préférence en une quantité allant de 0,000001 à 5 % en poids par rapport au poids total de la composition, et en particulier en une quantité allant de 0,0001 à 0,1 % en poids par rapport au poids total de la composition.

La composition topique selon l'invention peut se présenter notamment sous forme de solutions aqueuses, hydroalcooliques ou huileuses, ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, de microémulsions, ou encore de microcapsules, de microparticules, ou de dispersions vésiculaires de type ionique et/ou non ionique. Elles peuvent être aussi conditionnées sous forme de composition pour aérosol contenant également un agent propulseur sous pression. Ces compositions sont préparées selon les méthodes usuelles.

Elle peut être également utilisée pour le cuir chevelu sous forme de solutions aqueuses, alcooliques ou hydroalcooliques, ou sous forme de crèmes, de gels, d'émulsions, de mousse, ou encore de composition pour aérosol.

Les quantités des différents constituants des compositions selon l'invention sont celles classiquement utilisées dans les domaines considérés.

Ces compositions constituent notamment des crèmes de protection, de traitement ou de soin pour le visage, pour les mains, pour les pieds, pour les grands plis anatomiques ou pour le corps, des laits corporels de protection ou de soin, des lotions, gels ou mousses pour le soin de la peau et des muqueuses, comme des lotions de nettoyage ou de désinfection, des compositions pour le bain, des compositions contenant un agent bactéricide.

Les compositions peuvent également consister en des préparations solides constituant des savons ou des pains de nettoyage.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines considérés, tels que les gélifiants hydrophiles ou lipophiles, les actifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines, les extraits végétaux, notamment d'Aloe Vera, et les hydroxyacides.

Comme actifs lipophiles, on peut utiliser le tocophérol (vitamine E) et ses dérivés, le rétinol (vitamine A) et ses dérivés, les acides gras essentiels, les céramides, les huiles essentielles.

On peut également associer les antagonistes de substance P à des agents actifs, notamment des décongestionnants, des antibactériens, des antiseptiques et des antimicrobiens.

Parmi ces agents actifs, on peut citer à titre d'exemple :
- les antiseptiques tels que l'acide salicylique et ses dérivés (acide n-octanoyl-5-salicylique), ou le crotamiton.
- les antibactériens tels que le phosphate de clindamycine, l'érythromycine ou les antibiotiques de la classe des tétracyclines ;
- les antiparasitaires, en particulier le métronidazole ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique;
- les agents anesthésiques tels que le chlorhydrate de lidocaïne et ses dérivés ;
- les agents antiprurigineux comme la thénaldine, la triméprazine ou la cyproheptadine;
- les agents anti-radicaux libres, tels que l'alpha-tocophérol ou ses esters, les superoxyde dismutases, certains chélatants de métaux ou l'acide ascorbique et ses esters ;
- les agents kératolytiques comme l'acide rétinoïque 13 cis ou all trans, le peroxyde de benzoyle ou les hydroxyacides.

Les exemples suivants illustrent l'invention. Dans ces exemples, les proportions indiquées sont des pourcentages en poids.

| ***Exemple 1 : Crème pour le visage (émulsion huile dans eau)*** | |
|---|---|
| Spantide II | 0,3 % |
| Stéarate de glycérol | 2 % |
| Polysorbate 60 (Tween 60 vendu par la société ICI) | 1 % |
| Acide stéarique | 1,4 % |
| Triéthanolamine | 0,7 % |
| Carbomer | 0,4 % |
| Cyclométhicone | 8 % |
| Huile de tournesol | 12 % |
| Antioxydant | 0,05 % |
| Conservateur | 0,3 % |
| Eau | qsp 100 % |

| ***Exemple 2 : Gel émulsionné (émulsion huile* dans *eau)*** | |
|---|---|
| Cyclométhicone | 3,00 |
| Huile de Purcellin (vendue par la Société Dragocco) | 7,00 |
| PEG-6/PEG-32/Glycol Stéarate (Tefose^{R} 63 de | |
| Gattefosse) | 0,30 |
| N,N'-bis-di-(3,5-dimethoxybenzyle)-éthylène diamine | 0,5 |
| Conservateur | 0,30 |
| Carbomer | 0,60 |
| Crotamiton | 5,00 |
| Acide glycyrrhétinique | 2,00 |
| Alcool éthylique | 5,00 |
| Triéthanolamine | 0,20 |
| Eau | qsp 100% |

| ***Exemple 3 : Gel*** | |
|---|---|
| Acide all trans rétinoïque | 0,05 % |
| N,N'-bis-di-(3,5-dimethylbenzyle)- éthylène diamine | 5 % |
| Hydroxypropylcellulose (Klucel H vendu par la société Hercules) | 1 % |
| Antioxydant | 0,05 % |
| Isopropanol | 40 % |
| Conservateur | 0,3 % |
| Eau | qsp 100% |

| ***Exemple 4 : Crème (émulsion huile* dans eau)** | |
|---|---|
| Spantide II | 0,25 |
| Stéarate de glycérol | 2,00 |
| Polysorbate 60 (Tween 60 vendu par la société ICI) | 1,00 |
| Acide stéarique | 1,40 |
| Métronidazole | 0,50 |
| Triéthanolamine | 0,70 |
| Carbomer | 0,40 |
| Cyclométhicone | 8,00 |
| Huile de tournesol | 10,00 |
| Antioxydant | 0,05 |
| Conservateur | 0,30 |
| Eau | qsp 100% |

## Revendications

1. Utilisation d'un antagoniste de substance P pour la préparation d'une composition topique pharmaceutique ou dermatologique, destinée à traiter la rosacée.

2. Utilisation selon la revendication 1, **caractérisée en ce que** l'antagoniste de substance P est choisi parmi les peptides, les composés comprenant au moins un hétérocycle et les composés azotés comprenant un ou plusieurs cycles benzèniques.

3. Utilisation selon l'une quelconque des revendications 1 ou 2, **caractérisée en ce que** le peptide est le sendide ou le spantide II.

4. Utilisation selon l'une quelconque des revendications 1 ou 2, **caractérisée en ce que** le composé comprenant au moins un hétérocycle est un composé hétérocyclique azoté choisi parmi les dérivés de 2-tricyclyl-2-amino-éthane, les dérivé de spirolactame, les dérivés de quinuclidine, les dérivés azacycliques, les dérivés d'aminopyrrolidine, les dérivés de pipéridine, les aminoazahétérocycles, les dérivés d'isoindole.

5. Utilisation selon l'une quelconque des revendications 1 ou 2, **caractérisée en ce que** le composé comprenant au moins un hétérocycle est un composé hétérocyclique oxygéné ou soufré choisi parmi les dérivés du furanne, les dérivés du benzofuranne, les dérivés du thiophène et les dérivés du benzothiophène, et notamment les tétrazolyl-benzofuranne-carboxamides ou les tétrazolyl-benzothiophène-carboxamides.

6. Utilisation l'une quelconque des revendications 1 ou 2, **caractérisée en ce que** le composé azoté comprenant un ou plusieurs cycles benzèniques est un dérivé d'éthylène diamine.

7. Utilisation selon l'une quelconque des revendications précédentes, **caractérisée en ce que** l'antagoniste de substance P est utilisé en une quantité allant de 0,000001 à 5 % en poids par rapport au poids total de la composition.

8. Utilisation selon l'une quelconque des revendications précédentes, **caractérisée en ce que** l'antagoniste de substance P est utilisé en une quantité allant de 0,0001 à 0,1 % en poids par rapport au poids total de la composition.

9. Utilisation selon l'une quelconque des revendications précédentes, **caractérisée en ce que** la composition contient en outre au moins un actif choisi parmi les protéines ou les hydrolysats de protéine, les acides aminés, les polyols, l'urée, les sucres et les dérivés de sucre, les vitamines, l'amidon, les extraits végétaux, les hydroxyacides, les céramides, les huiles essentielles.

10. Utilisation selon l'une quelconque des revendications précédentes, **caractérisée en ce que** la composition contient en outre au moins un agent choisi parmi les agents antiseptiques, antibactériens, antiparasitaires, antifongiques, anti-inflammatoires, antiprurigineux, anesthésiques, antiviraux, kératolytiques, antiradicaux libres.

11. Utilisation selon l'une quelconque des revendications précédentes, **caractérisée en ce que** la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

## Claims

1. Use of a substance P antagonist for the preparation of a topical pharmaceutical or dermatological composition intented for treating acne rosacea.

2. Use according to Claim 1, **characterized in that** the substance P antagonist is chosen from peptides, compounds comprising at least one heterocycle and nitrogen-containing compounds comprising one or more benzene rings.

3. Use according to any one of Claims 1 to 2, **characterized in that** the peptide is sendide or spantide II.

4. Use according to any one of Claims 1 to 2, **characterized in that** the compound comprising at least one heterocycle is a nitrogen-containing heterocyclic compound chosen from 2-tricyclyl-2-aminoethane derivatives, spirolactam derivatives, quinuclidine derivatives, azacydic derivatives, aminopyrrolidine derivatives, piperidine derivatives, aminoazaheterocycles, isoindole derivatives.

5. Use according to any one of Claims 1 to 2, **characterized in that** the compound comprising at least one heterocycle is an oxygen-containing or sulphur-containing heterocyclic compound chosen from furan derivatives, benzofuran derivatives, thiophene derivatives and benzothiophene derivatives, and especially tetrazolylbenzofuran-carboxamides or tetrazolylbenzothiophene-carboxamides.

6. Use according to any one of Claims 1 to 2, **characterized in that** the nitrogen-containing compound comprising one or more benzene rings is an ethylenediamine derivative.

7. Use according to any one of the preceding claims, **characterized in that** the substance P antagonist is used in a quantity ranging from 0.000001 to 5 % by weight relative to the total weight of the composition.

8. Use according to any one of the preceding claims, **characterized in that** the substance P antagonist is used in a quantity ranging from 0.0001 to 0.1 % by weight relative to the total weight of the composition.

9. Use according to any one of the preceding claims, **characterized in that** the composition contains, in addition, at least one active agent chosen from proteins, protein hydrolysates, amino acids, polyols, urea, sugars and sugar derivatives, vitamins, starch, plant extracts, hydroxy acids, ceramides, essential oils.

10. Use according to any one of the preceding claims, **characterized in that** the composition contains, in addition, at least one agent chosen from antiseptic, antibacterial, antiparasitic, antifungal, antiinflammatory, antipruritic, anaesthetic, antiviral, keratolytic and anti-free-radical agents.

11. Use according to any one of the preceding claims, **characterized in that** the composition is an aqueous, oily or aqueous-alcoholic solution, a water-in-oil emulsion, an oil-in-water emulsion, a microemulsion, an aqueous gel, an anhydrous gel, a serum, a dispersion of vesicles, of microcapsules or of microparticles.

## Patentansprüche

1. Verwendung eines Antagonisten der Substanz P zur Herstellung einer topischen pharmazeutischen oder dermatologischen Zusammensetzung zur Behandlung von Rosacea vorgesehen ist.

2. Verwendung nach Anspruch 1, **dadurch gekennzeichnet, daß** der Antagonist der Substanz P unter den Peptiden, den Verbindungen, die mindestens einen Stickstoffheterocyclus aufweisen, und den Stickstoffverbindungen, die einen oder mehrere Benzolringe enthalten, ausgewählt ist.

3. Verwendung nach einem der Ansprüche 1 bis 2, **dadurch gekennzeichnet, daß** das Peptid Sendide oder Spantide II ist.

4. Verwendung nach einem der Ansprüche 1 bis 2, **dadurch gekennzeichnet, daß** die Verbindung die mindestens einen Stickstoffheterocyclus aufweist, eine heterocyclische Stickstoffverbindung ist, die unter den 2-Tricyclyl-2-aminoethanderivaten, Spirolactamderivaten, Chinuclidinderivaten, azacylischen Derivaten, Aminopyrrolidonderivaten, Piperidinderivaten, Aminoazaheterocyclen und Isoindolderivaten ausgewählt ist.

5. Verwendung nach einem der Ansprüche 1 bis 2, gekennzeichnet, daß die Verbindung, die mindestens einen Heterocyclus enthält, eine heterocyclische Sauerstoff- oder Schwefelverbindung ist, die unter den Furanderivaten, Benzofuranderivaten, Thiophenderivaten, Benzothiopherrierivaten und insbesondere Tetrazolyl-benzofuran-carboxamiden oder Tetrazolyl-benzothiophen-carboxamiden ausgewählt ist.

6. Verwendung nach einem der Ansprüche 1 bis 2, **dadurch gekennzeichnet, daß** die Stickstoffverbindung, die einen oder mehrere Benzolringe enthält, ein Ethylendiaminderivat ist.

7. Verwendung nach einem der vorhergehenden Ansprüche, **dadurch gekennzeichnet, daß** der Antagonist der Substanz P in einen Mengenanteil von 0,000001 bis 5 Gew.-%, bezogen auf das Gesamtgewicht der Zusammensetzung, verwendet wird.

8. Verwendung nach einem der vorhergehenden Ansprüche, **dadurch gekennzeichnet, daß** der Antagonist der Substanz P in einem Mengenanteil von 0,0001 bis 0,1 Gew.-%, bezogen auf das Gesamtgewicht der Zusammensetzung, verwendet wird.

9. Verwendung nach einem der vorhergehenden Ansprüche, **dadurch gekennzeichnet, daß** die Zusammensetzung ferner mindestens einen Wirkstoff enthält, der unter den Proteinen oder Proteinhydrolysaten, Aminosäuren, Polyolen, Harnstoff, Zuckern und Zuckerderivaten, Vitaminen, Stärke, pflanzlichen Extrakten, Hydroxysäuren, Ceramiden und etherischen Ölen ausgewählt ist.

10. Verwendung nach einem der vorhergehenden Ansprüche, **dadurch gekennzeichnet, daß** die Zusammensetzung ferner mindestens ein Mittel enthält, das unter den antiseptischen Mitteln, antibakteriellen Mitteln, Mitteln gegen Parasiten, Mitteln gegen Pilze, Antiphlogistika, Antipruriginosa, Anästhetika, Mitteln gegen Viren, Keratolytika und Mitteln gegen freie Radikale ausgewählt ist.

11. Verwendung nach einem der vorhergehenden Ansprüche, **dadurch gekennzeichnet, daß** die Zusammensetzung eine wäßrige, ölige oder wäßrig-alkoholische Lösung, eine Wasser-in-Öl-Emulsion, eine Öl-in-Wasser-Emulsion, eine Microemulsion, ein wäßriges Gel, ein wasserfreies Gel, ein Serum, eine Vesikeldispersion oder Dispersion von Microkapseln oder Micropartikeln ist.
